(19) Europäisches Patentamt
European Patent Office
Office européen des brevets

(11) Publication number: 0 061 162
A2

# (12) EUROPEAN PATENT APPLICATION

(21) Application number: 82102243.1

(22) Date of filing: 19.03.82

(51) Int. Cl.³: C 07 D 501/00
A 61 K 31/545

(30) Priority: 23.03.81 JP 42718/81
01.10.81 JP 156466/81
30.10.81 JP 175388/81
15.11.81 JP 182600/81

(43) Date of publication of application:
29.09.82 Bulletin 82/39

(84) Designated Contracting States:
AT BE CH DE FR GB IT LI NL SE

(71) Applicant: KYOTO YAKUHIN KOGYO KABUSHIKI KAISHA
38, Nishinokyo Tsukinowa-cho Nakakyo-ku
Kyoto(JP)

(72) Inventor: Nobuharu, Kakaya
3-10-16 Takadai Nagaokakyo
Kyoto(JP)

(72) Inventor: Kazuhiko, Kitao
12 Sandan Osa-cho Matsugasaki
Sakyo-ku Kyoto(JP)

(74) Representative: von Kreisler, Alek, Dipl.-Chem. et al,
Deichmannhaus am Hauptbahnhof
D-5000 Köln 1(DE)

(54) **Cephalosporin derivatives, pharmaceutical compositions containing the same and production thereof.**

(57) Novel cephalosporin derivatives represented by the general formula

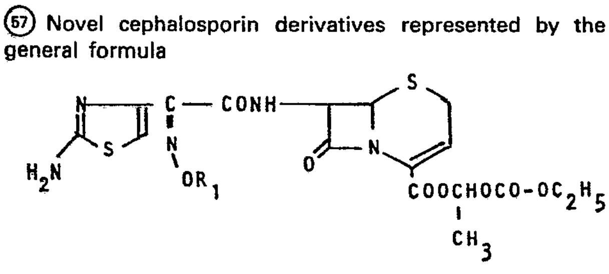

wherein $R_1$ is a lower alkyl group, and acid addition salts thereof are found to be useful as orally administrable antibiotics having broad antimicrobial activities against both gram-positive and gram-negative bacteria.

EP 0 061 162 A2

CEPHALOSPORIN DERIVATIVES, PHARMACEUTICAL COMPOSITIONS CONTAINING THE SAME, AND PRODUCTION THEREOF

This invention relates to cephalosporin derivatives represented by the general formula

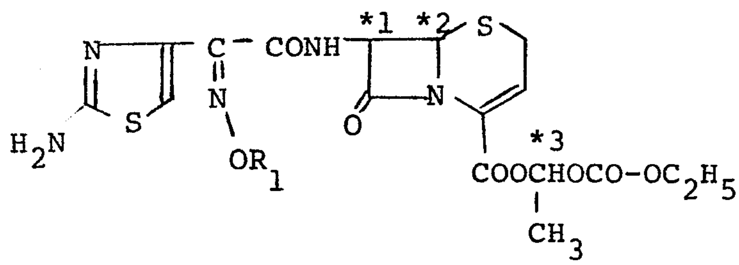

(I)

wherein $R_1$ is a lower alkyl group, and acid addition salts thereof, pharmaceutical compositions containing said cephalosporin derivatives or acid addition salts thereof as active ingredients, methods of producing said cephalosporin derivatives, and methods of producing said pharmaceutical compositions.

Cephalosporins in general are poorly absorbed through the digestive tract and therefore are administered by injection.

As a result of intensive research to find out orally administrable cephalosporin derivatives, the present inventors have found (1) that the above-mentioned novel cephalosporin derivatives (I) are absorbable through the digestive tract to a satisfactory extent and can rapidly be hydrolyzed in vivo under the action of enzymes, whereby the ester moiety of the substituent at the 4-position of the cephem ring structure is hydrolyzed and cephalosporin derivatives (I) are thereby converted to the corresponding unesterified species (namely the compounds having a free carboxyl group at the 4-position), namely that oral administration of cephalosporin derivatives (I) results in high blood levels of those unesterified species that have an excellent antibacterial activity, which high blood levels can be retained for a

prolonged period of time, (2) that conversion of cephalosporin derivatives (I) into acid addition salts thereof results in improved absorbability and at the same time in stabilization of cephalosporin derivatives (I) and facilitation of the isolation procedure and of the production of pharmaceutical preparations for oral administrations, (3) that cephalosporin derivatives (I) and acid addition salts thereof in the crystalline hydrate form, especially the crystalline hydrate of the hydrochloride of that compound of general formula (I) in which $R_1$ is methyl, are excellent in stability and therefore are more preferable as drugs, (4) that specific epimers of said cephalosporin derivatives (I) are superior in absorbability through the digestive tract, and (5) that, when cephalosporin derivatives (I) or acid addition salts thereof are administered orally in the presence of organic acids, the absorbability of cephalosporin derivatives (I) or acid addition salts thereof is much increased. The present invention has been completed on the basis of these findings.

Thus, the invention provides

(1) cephalosporin derivatives (I) and acid addition salts thereof,

(2) orally administrable pharmaceutical compositions for the treatment of infectious diseases caused by bacteria, which compositions contain at least one member selected from the group consisting of cephalosporin derivatives (I) and acid addition salts thereof,

(3) a method of producing cephalosporin derivatives (I) or acid addition salts thereof, which comprises reacting a compound of the general formuls (II) shown hereinafter with a compound of the general formula (III) shown hereinafter or reacting a compound of the general formula (IV) shown hereinafter with a compound of the general formula (V) shown hereinafter, and

(4) a method of preparing orally administrable pharmaceutical compositions for the treatment of infectious diseases caused by bacteria, which comprises uniformly mixing a cephalosporin derivative (I) with an organic acid.

Referring to general formula (I), the lower alkyl group represented by $R_1$ may be either straight-chained or branched and preferably contains 1-4 carbon atoms. Thus, preferred examples are methyl, ethyl, n-propyl, iso-propyl, n-butyl, iso-butyl and tert-butyl.

Cephalosporin derivatives (I) may occur as hydrates, as the case may be. Such hydrates are covered by the present invention. Preferred hydrates are hemihydrate to trihydrate, especially hemihydrate and monohydrate.

It is preferable that cephalosporin derivatives (I) are in the acid addition salt form on the part of the aminothiazole moiety thereof. Any pharmaceutically acceptable acids which can form salts with said aminothiazole moiety may be used as acids for forming said acid addition salts. Examples of such acids are such mineral acids as hydrochloric acid, sulfuric acid, phsophoric acid and nitric acid, and such organic acids as oxalic acid, fumaric acid, maleic acid, citric acid, tartaric acid, methanesulfonic acid and toluenesulfonic acid.

Among said acid addition salts preferred is the hydrochloride, especially in the form of a crystalline hydrate, preferably hemihydrate or monohydrate.

Whereas cephalosporin derivatives (I) each includes syn and anti geometric isomers with respect to the oxyimino moiety thereof, the syn form is preferred in the practice of the present invention.

The aminothiazole moiety of cephalosporin derivatives (I) may take the iminothiazoline form, which is a tautomeric form, as the case may be. It goes without saying that such embodiment is also covered by the present invention.

Cephalosporin derivatives (I) each contains three asymmetric carbon atoms marked with *1, *2 and *3 in general

formula (I) and consequently includes several optical isomers.

An advanced research made on these compounds by the present inventors has successfully led to

(1) isolation of two optically active species of a compound represented by the formula

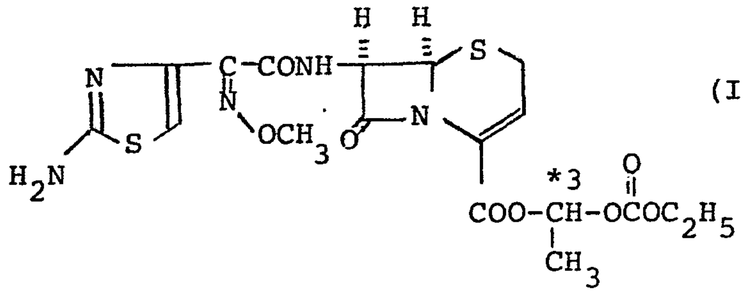

(I')

which species are due to the asymmetric carbon atom marked with *3, one species (hereinafter "Epimer A") having the specific rotation of $[\alpha]_D^{26}$ +48° (c=1, methanol) and the other (hereinafter "Epimer B") having the specific rotation of $[\alpha]_D^{26}$ +113° (c=1, methanol), and acid addition salts of each, and has revealed

(2) that acid addition salts of Epimer A are more excellent in absorbability through the digestive tract and at the same time more stable and

(3) that acid addition salts of Epimer A may occur as crystalline hydrates (e.g. hemihydrate and monohydrate), which have good stability and therefore may preferably be used as drugs.

The present invention also covers such Epimer A and Epimer B as well as acid addition salts thereof.

Cephalosporin derivatives (I) can be produced, for example, by the following method. Thus, they can be produced by reacting a compound of the general formula

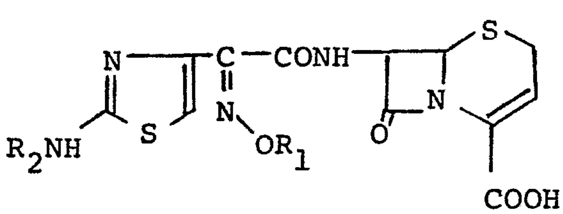

(II)

wherein $R_1$ is as defined above and $R_2$ is H or amino-protecting group, or a reactive derivative thereof with a compound of the general formula

$$X-\underset{\displaystyle CH_3}{\underset{|}{CH}}-O-\underset{\displaystyle O}{\underset{\|}{C}}-OC_2H_5 \qquad (III)$$

wherein X is an atom or group reactive with a carboxylic acid or a reactive derivative thereof, eliminating the protective group $R_2$ if $R_2$ is such.

Reffering to X in general formula (III), said reactive atom or group is, for example, a halogen atom or a group of the formula $B-SO_3-$ (B being an alkyl or substituted aryl group). The alkyl group represented by B is preferably a lower alkyl group containing 1-4 carbon atoms, such as methyl, ethyl, n-propyl or n-butyl, and the substituted aryl group is preferably a lower $(C_{1-4})$ alkyl-substituted phenyl group, such as p-tolyl. Examples of said halogen atom are chlorine, bromine and iodine. Reffering to $R_2$ in general formula (II), any protective groups which can be removed when necessary may be used. Examples of the protective group which are usable in many cases are 2,2,2-trichloroethoxycarbonyl, 2-methylsulfonylethyloxycarbonyl, tert-butoxycarbonyl, chloroacetyl and trityl.

Preferably, compound (II) is subjected to the reaction in the form of a reactive derivative although compound (II) may be used as it is. The reactive derivative of compound (II) is a reactive derivative with respect to the carboxyl group thereof and includes reactive derivatives commonly used in esterification reactions, such as inorganic salts, for example alkali metal salts (e.g. sodium salt, potassium salt), and organic salts, for example organic amine salts (e.g. triethylamine salt).

The reaction of compound (II) in the salt form with compound (III) is preferably carried out under cooling so as to avoid

formation of the byproduct $\Delta^2$-isomer.

The reaction can easily be carried out in the presence of a solvent which does not interfere with the reaction, such as dimethylformamide, dimethylacetamide, hexamethylphosphorotriamide, acetone or acetonitrile. After the esterification reaction, the protective group is eliminated as necessary from the protected amino group by a conventional method known *per se* to give cephalosporin derivatives (I).

Compounds (III) are generally known compounds and can be prepared by conventional method.

Cephalosporin derivatives (I) can also be produced, for example, by reacting a compound of the general formula

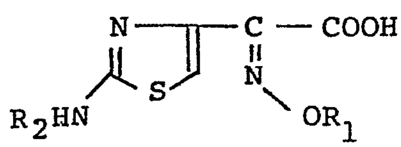

(IV)

wherein $R_1$ and $R_2$ are as defined above, or a reactive derivative thereof with a compound of the general formula

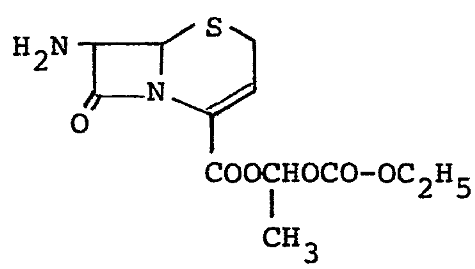

(V)

eliminating the protective group $R_2$ if $R_2$ is such.

Compound (IV) is used in the free carboxylic acid form or as a reactive derivative thereof. Thus, the free acid or such a reactive derivative thereof as a salt (e.g. sodium, potassium, calcium, triethylamine or pyridine salt), an acid halide (e.g. acid chloride, acid bromide), an acid anhydride, a mixed acid anhydride [e.g. with a substituted phosphoric acid (e.g. dialkylphosphoric acid) or an alkyl carbonic acid

(e.g. monoethyl carbonic acid)], an active amide (e.g. amide with imidazole) or an ester (e.g. cyanomethyl ester, 4-nitrophenyl ester) is subjected to the acylation.

In cases where compound (IV) is used in the free acid or salt form, the use of an adequate condensing agent is preferable and usable condensing agents are, for example, such dehydrating agents as N,N'-disubstituted carbodiimides (e.g. N,N'-dicyclohexylcarbodiimide) and azolide compounds (e.g. N,N'-carbonyldiimidazole, N,N'-thionyldiimidazole). When such a condensing agent is used, the reaction presumably proceed via a reactive derivative of the carboxylic acid.

The reaction is carried out generally in an inert solvent. Examples of the solvent are water, acetone, dioxane, acetonitrile, chloroform, benzene, methylene chloride, ethylene chloride, tetrahydrofuran, ethyl acetate, N,N-dimethylformamide, pyridine, other organic solvents, and mixtures of these.

The reaction is preferably carried out at room temperature or under cooling (-20°C to 0°C).

Compound (V) can be prepared, for example, by reacting a compound of the general formula

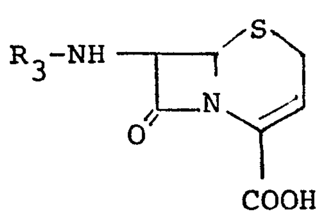

(VI)

wherein $R_3$ is H or an amino-protecting group, or a reactive derivative thereof with compound (III).

Referring to $R_3$ in general formula (VI), the amino-protecting group includes *per se* known amino-protecting groups such as benzyloxycarbonyl, 2-thienylacetyl, 2-furylacetyl, D-5-amino-5-carboxyvaleryl, trityl and phthalimido.

It is preferable that compound (VI) is subjected to the reaction as a reactive derivative thereof (e.g. alkali metal salt, such as sodium salt or potassium salt, alkaline earth metal salt, such as calcium salt, triethylamine salt,

pyridine salt).

For preventing formation of the byproduct $\Delta^2$-isomer, this reaction is preferably carried out under cooling. The presence of a solvent which does not interfere with the reaction (e.g. dimethylformamide, dimethylacetamide, hexamethylphosphorotriamide, acetone, acetonitrile) may facilitate the progress of the reaction.

It is preferable in carrying out the reaction that $R_3$ in general formula (VI) is an amino-protecting group. In this case, the reaction of compound (VI) with compound (III) gives compound (V) in which the amino group in the 7-position is protected. The protective group can be eliminated by a per se known method of eliminating protective groups.

As the method of eliminating said protective group, there may be mentioned, for removal of benzyloxycarbonyl, 2-thienylacetyl, 2-furylacetyl and D-5-amino-5-carboxyvaleryl, for instance, the method comrising iminochlorination with phosphorus pentachloride, followed by decomposition with methanol, and for elimination of trityl, for instance, the method comprising treatment with an acid (e.g. formic acid, trifluoroacetic acid), and for removal of phthalimido, for instance, Ing-Manske's method using hydrazine.

The thus-obtained cephalosporin derivatives (I) can be converted into acid addition salts thereof by a per se known method.

The crystalline hydrate of the hydrochloride of cephalosporin derivative (I) can be prepared, for example, by dissolving cephalosporin derivative (I) in an aqueous hydrochloric acid-containing solution or the hydrochloride of cephalosporin derivative (I) in an aqueous solvent or an aqueous hydrochloric acid-containing solution and then causing the crystalline hydrate of the hydrochloride of compound (I) to crystallize out.

As the aqueous solvent, there may be used water or a mixture of water and an organic solvent such as an alcohol

(e.g. methanol, ethanol, propanol), acetone, dioxane or tetrahydrofuran.

As the aqueous hydrochloric acid-containing solvent, there may be used aqueous hydrochloric acid or a mixture of aqueous hydrochloric acid and an organic solvent such as mentioned above.

Referring to the aqueous hydrochloric acid-containing solvent to be used in producing the crystalline hydrate, the aqueous hydrochloric acid generally has the hydrochloric acid concentration of 0.01% to 20%, preferably 0.1% to 10%.

The dissolution of cephalosporin derivative (I) or the acid addition salt thereof in an aqueous solvent or an aqueous hydrochloric acid-containing solvent is generally effected at 10°C to 70°C, preferably at 20°C to 50°C.

The hydrate is caused to crystallize out, for example, by (1) concentrating under reduced pressure (preferably at room temperature) to an adequate volume or (2) dissolving with heating and then cooling or (3) adding an adequate amount (e.g. 5% to 15%) of sodium chloride, filtering off the insoluble matter and allowing the filtrate to stand and cool.

The thus-formed crystalline precipitate of the hydrate can be collected by a <u>per se</u> known means, for example by filtration. When the thus-obtained crystalline hydrate is dried sufficiently and submitted to elemental analysis, the analytical data indicate that the product is the hydrate. Observation under a polarization microscope and X-ray diffraction pattern ascertain that the product is crystalline. Furthermore, the infrared absorption spectrum of the product is different from that of the amorphous hydrochloride of cephalosporin derivative (I). The former spectrum presents sharp peaks.

Epimer A, Epimer B, and acid addition salts thereof are prepared, for instance, by the following procedures:

<u>Procedure 1</u>

One weight part of that species of cephalosporin derivative (I) which is optically inactive with respect to the asymmetric carbon atom marked with *3 (hereinafter "optically inactive compound (I)") or the hydrochloride thereof is dissolved in 20 to 120 weight parts of 0.1-2% aqueous hydrochloric acid with heating and then allowed to crystallize out, whereupon Epimer B in the hydrochloride form crystallizes out. Epimer A hydrochloride remains in the mother liquor and can be recovered in the crude form, for instance by concentrating the mother liquor. The crude Epimer A hydrochloride is recrystallized from 40-60 times its weight of 0.1-0.5% aqueous hydrochloric acid to give purified Epimer A hydrochloride.

Epimer A and Epimer B each in the hydrochloride form, which has crystallized out, can be collected by a per se known means, for example by filtration.

Generally, the Epimer A hydrochloride obtained by this procedure occurs as hemihydrate crystals of the hydrochloride whereas the Epimer B hydrochloride occurs as monohydrate crystals of the hydrochloride.

When such hydrates are dissolved in a polar solvent such as ethyl acetate and then a non-polar solvent such as isopropyl ether is added, the respective epimers can be recovered each as a solid precipitate in the non-hydrated form.

Procedure 2

One weight part of an acid addition salt especially hydrochloride of optically inactive compound (I)" is dissolved in 20 weight parts of 95% ethanol with heating and then caused to crystallize out, whereupon Epimer B in the acid addition salt form crystallizes out. Epimer A in the acid addition salt form remains in the mother liquor and can be recovered in the crude form, for example by concentrating the mother liquor. The crude acid addition salt of Epimer A can be purified by recrystallization from 10 times its weight of 95% ethanol.

Procedure 2 is suited for preparing Epimer A hydrochloride and Epimer B hydrochloride. The thus-obtained Epimer A hydrochloride generally occurs as hymihydrate crystals and

the Epimer B hydrochloride generally occurs as monohydrate crystals.

Procedure 3

Epimer A can be prepared by reacting a compound of the formula

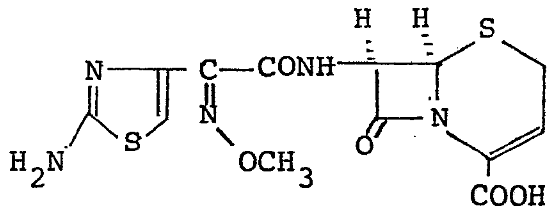

(II')

with an optically active isomer of compound (III).

Procedure 4

Epimer A can be isolated by recrystallizing cephalosporin derivative (I) from an organic solvent.

The organic solvent includes, among others, alcohols such as methanol and ethanol, ethers such as diethyl ether and isopropyl ether, acetone, chloroform, and mixtures of these.

The thus-obtained acid addition salt of Epimer A or B can be converted to Epimer A or B having the free amino group by neutralization with sodium hydrogen carbonate, for instance. Conversely, Epimer A or B having the free amino group can be converted to an acid addition salt by a *per se* known means.

The orally administrable therapeutic compositions for treating infectious diseases caused by bacteria in accordance with the present invention can be prepared by diluting the thus-produced cephalosporin derivatives (I) or acid addition salts thereof with a pharmaceutical carrier or excipient by a *per se* known procedure. The dilution is carried out in a conventional manner, for example by mixing. The carrier or excipient includes starch, lactose, sucrose, calcium carbonate and calcium phosphate, among others.

In accordance with the present invention, it is preferable to add an organic acid, preferably an aliphatic carboxylic acid to the pharmaceutical composition. The addition of

such acid facilitates absorption of cephalosporin derivatives (I) and acid addition salt thereof into the blood circulation. Any organic acid, especially aliphatic carboxylic acid species that are pharmaceutically acceptable may be used. Generally, aliphatic carboxylic acid having 1-4 carboxyl groups, preferably 2-3 carboxyl groups, and optionally having a hydroxyl group or hydroxyl groups are used. The aliphatic carboxylic acid thus includes tartaric acid, malic acid, glucuronic acid, citric acid, maleic acid, fumaric acid, etc.

The advantages of solid preparations containing cephalosporin derivatives (I) or acid addition salts thereof, such as tablets, capsules, granules and pills, as compared with liquid preparations containing the same are that the former preparations are more convenient to handle and that cephalosporin derivatives (I) and acid addition salts thereof are more stable in the former preparations. However, it has been found that, when made into solid preparations, cephalosporin derivatives (I) and acid addition salts thereof are poor in absorbability after oral administration as compared with liquid preparations. When the above-mentioned organic acid is added to such solid preparations, solid preparations excellent in absorbability after oral administration are obtained. Therefore, the significance of the above-mentioned organic acid becomes apparent when it is added to such solid preparations.

Said organic acid is used in the therapeutic compositions for treating bacteria-caused infectious diseases in accordance with the invention in an amount sufficient to increase absorption of cephalosporin derivatives (I) after oral administration. The amount depends upon the kind of cephalosporin derivative (I) and other factors. Generally, it is 0.1-20 moles, preferably 1-10 moles, per mole of cephalosporin derivative (I) or an acid addition salt thereof, or generally 5-500 mg, preferably 10-250 mg, per dose.

The bacteria-caused infectious disease-treating compositions according to the invention may contain other

additives, if desirable. Preferred examples of such additives are binders (e.g. starch, gum arabic, carboxymethylcellulose, hydroxypropylcellulose, crystalline cellulose), lubricants (e.g. magnesium stearate, talc) and disintegrators (e.g. carboxymethylcellulose, talc). The solid preparations may also contain alkali metal carbonates (e.g. sodium carbonate, potassium carbonate), alkali metal hydrogen carbonates (e.g. sodium hydrogen carbonate, potassium hydrogen carbonate), etc. After mixing the ingredients, the mixture can be formed into dosage forms suited for oral administration, such as capsules, powder, granules and dry sirup, by per se known means.

When the bacteria-caused infectious disease-treating compositions according to the invention are administered orally, the active ingredients, namely cephalosporin derivatives (I) or acid addition salts thereof, are rapidly absorbed through the digestive tract and, immediately after absorption, are hydrolyzed in vivo under the action of enzymes to give the corresponding unesterified cephalosporins or salts thereof.

Said unesterified cephalosporins and salts thereof have an excellent antibacterial activity. They are markedly active against such gram-positive bacteria as Staphylococcus aureus and such gram-negative bacteria as Escherichia coli, Klebsiella pneumoniae, Proteus vulgaris, Proteus mirabilis and Proteus morganii. Moreover, the unesterified cephalosporins and salts thereof are very low in toxicity.

Therefore, the bacteria-caused infectious disease-treating compositions according to the present invention can be used as therapeutic agents, for example for bacteria-caused diseases (e.g. suppurative diseases, respiratory tract infection, biliary tract infection, urinary tract infection) in humans and warm-blooded animals (e.g. dog, cat, cattle, horse, rat, mouse).

The dose of cephalosporin derivatives (I) or salts thereof depends on subjects, severity of symptom and other factors. In treating suppurative diseases in human adults,

for instance, cephalosporin derivatives (I) or salts thereof are administered orally at a dose of about 1-20 mg (as the corresponding unesterified cephalosporins) per kg body weight three or four times per day.

## Reference Example 1

Preparation of 1-(ethoxycarbonyloxy)ethyl 7-(phenylacetamido)-3-cephem-4-carboxylate

To a solution of 5.0 g of potassium 7-(phenylacetamido)-3-cephem-4-carboxylate in 100 ml of dimethylformamide was added 5.1 g of ethyl 1-iodoethyl carbonate with ice cooling (-20°C to -10°C) and stirring. The mixture was stirred for 0.5-1 hour. Then, 800 ml of ethyl acetate was added and the mixture washed with water ( 300 ml x 3) and then with a saturated aqueous sodium chloride solution (100 ml x 2) and dried over sodium sulfate. Concentration of the solution and addition of 100 ml of isopropyl ether gave 3.5 g of the title compound as a white crystalline powder.

IR (Nujol, $cm^{-1}$): 1775, 1750, 1740, 1680

NMR ($(CD_3)_2CO$, ppm): 1.25 (t, J=7Hz, 3H, $-CH_2-CH_3$), 1.52 (d, J=5Hz, 3H, $-\overset{|}{C}H-CH_3$), 3.4~3.75 ( m, 2H, $S-CH_2$), 3.6 (s, 2H, 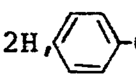$-CH_2-$), 4.15 (q, J=7Hz, 2H, $-CH_2CH_3$), 4.96 (d, J=5Hz, 1H, 6-H), 5.82 (dx d, J=5 and 9Hz, 1H, 7-H), 6.15 (d x d, J=5.5 and 3Hz, 1H, 3-H) 6.8 (q, J=5Hz, 1H, $-\overset{|}{C}H-CH_3$), 7.23 (s, 5H 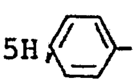-), 7.87 (d, J=9Hz, 1H, -CONH-)

## Reference Example 2

Preparation of p-toluenesulfonic acid salt of 1-(ethoxycarbonyloxy)ethyl 7-amino-3-cephem-4-carboxylate

In 250 ml of anhydrous methylene chloride was dissolved 4.3 g of the compound as prepared in Reference Example 1. The solution was cooled to -15°C, and thereto were added 9.7 ml of anhydrous pyridine and 80 ml of an 8% (w/v) solution of phosphorus pentachloride in methylene chloride. The mixture was stirred at -10°C to 5°C for an hour. Then, 67 ml of absolute methanol was added with caution not to allow the temperature to rise above -10°C. Thereafter stirring was continued under the same conditions for an hour, then the temperature was allowed to rise to room temperature over an hour, and 200 ml of 0.5 M $KH_2PO_4$ was added with vigorous stirring. The mixture was adjusted to pH 2.0 with 5 M $H_3PO_4$ and stirred at room temperature for 20 minutes. The organic layer was separated, the aqueous layer was extracted with two 130-ml portions of methylene chloride, and the above organic layer and the extract layers were combined, washed twice with water, dried over sodium sulfate and concentrated to dryness. The residue thus obtained was dissolved in 5 ml of ethyl acetate and thereto was added a solution of 2.8 g of p-toluenesulfonic acid in 38 ml of ethyl acetate. The subsequent concentration, addition of a small amount of methylene chloride and addition of ethyl ether yielded a solid precipitate. This was collected by filtration to give 3.2 g of the title compound.

IR (Nujol, $cm^{-1}$): 1790, 1755

NMR ($CDC\ell_3$, ppm): 1.24 (t (br), J=7Hz, 3H, $-CH_2CH_3$), 1.51 (d (br), J=5Hz, 3H, $-\overset{|}{C}HCH_3$), 2.3 (s (br), 3H, $CH_3$-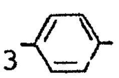-), 3.05~3.6 (m, 2H, $-SCH_2-$),

4.2 (q (br), J=7Hz, 2H, $-CH_2CH_3$), 4.77~5.05 (m, 1H, 6-H), 6.86 (q, J=5Hz, 1H, $-CHCH_3$), 7.05 (d, J=8Hz, 2H, 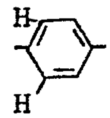$-SO_3-$), 7.73 (d, J=8Hz, 2H, 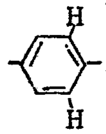$-SO_3$), 6.4~8.5 (br, $-NH_3$)

Synthesis Example 1

Preparation of 1-(ethoxycarbonyloxy)ethyl 7-[2-(2-aminothiazol-4-yl)-2-methoxyiminoacetamido]-3-cephem-4-carboxylate (syn isomer) [Compound 1]

To an ice-cooled and stirred solution of 1.0 g of sodium 7-[2-(2-aminothiazol-4-yl)-2-methoxyiminoacetamido]-3-cephem-4-carboxylate in 10 ml of dimethylformamide, there was added 0.5 g of ethyl 1-iodoethyl carbonate, and the mixture was stirred for 30 minutes. Thereafter, 80 ml of ethyl acetate was added and the mixture was washed with water (30 ml x 3) and then with a saturated aqueous sodium chloride solution (10 ml x 2) and dried over sodium sulfate. The solution was concentrated and 10 ml of isopropyl ether added to give 0.7 g of the title compound as a white crystalline powder.

IR (KBr, $cm^{-1}$): 3320, 1780, 1760

NMR ($(CD_3)_2CO$, ppm): 1.26 (t, 3H, J=7Hz, $-CH_3$), 1.55 (d, 3H, J=6Hz, $-CH_3$), 3.5~3.8 (m, 2H, $-SCH_2-$), 3.9 (s, 3H, $-OCH_3$), 4.17 (q, 2H, J=7Hz, $-CH_2-$), 5.17 (d, 1H, J=5Hz, $-CH-$), 6.01 (d.d, 1H, J=5Hz, J=9Hz, $-CH-$), 6.5~6.7 (m, 1H, =CH-), 6.5~7.5 (b, 2H, $-NH_2$), 6.76 (s, 1H, =CH-), 6.84 (q, 1H, $-CH-$), 8.42 (d, 1H, J=9Hz, -CONH- )

Synthesis Example 2

Preparation of Compound 1

To 26 ml of a 2.2% solution of phosphorus pentachloride in methylene chloride, there was added 1.27 g of 2-(2-tritylaminothiazol-4-yl)-2-methoxyiminoacetic acid at -20°C to -15°C with stirring. The mixture was stirred at the same temperature for 30 minutes. Thereafter 1.1 ml of triethylamine was added at -20°C, then a solution of 0.82 g of 1-(ethoxycarbonyloxy)-ethyl 7-amino-3-cephem-4-carboxylate as prepared in Reference Example 2 in 25 ml of methylene chloride was added all at once, and the temperature of the mixture was allowed to rise to room temperature over an hour with stirring. The reaction mixture, after addition of 100 ml of water, was extracted with two 120-ml portions of methylene chloride. The organic layer was washed with a saturated aqueous sodium hydrogen carbonate solution and dried over sodium sulfate, the methylene chloride then distilled off, and isopropyl ether added to the residue for crystallization thereof to give 1.5 g of 1-(ethoxycarbonyloxy)ethyl 7-[2-(2-tritylaminothiazol-4-yl)-2-methoxyiminoacetamido]-3-cephem-4-carboxylate (syn isomer). This compound (1.5 g) was dissolved in 15 ml of formic acid, and 5 ml of water added. The reaction was allowed to proceed at room temperature for 2 hours. The reaction mixture was then poured into an appropriate amount of water, the insoluble matter filtered off, and the filtrate made alkaline with sodium hydrogen carbonate and extracted with ethyl acetate. The organic layer was washed with a saturated aqueous sodium chloride solution and dried over sodium sulfate, the ethyl acetate distilled off under reduced pressure, and isopropyl ether added to the residue for crystallization thereof to give 0.8 g of the title compound.

Synthesis Example 3

The product compound of Synthesis Example 1 (0.5 g) was dissolved in 10 ml of ethyl acetate, and thereto were added 300 mg of 13% hydrogen chloride-in-isopropyl alcohol

solution and 4 ml of ethyl ether, whereupon a white crystalline powder precipitated. The powder was collected by suction filtration and dried under reduced pressure to give the title compound of Synthesis Example 1 in the hydrochloride form [Compound 2]. Yield 0.4 g.

IR (KBr, $cm^{-1}$): 1780, 1758

NMR ($(CD_3)_2SO$), ppm): 1.25 ( t, 3H, J=7Hz, $-CH_3$), 1.52 (d, 3H, J=6Hz, $-CH_3$), 3.46~3.86 (m, 2H, $-SCH_2-$), 3.96 (s, 3H, $-OCH_3$), 4.15 (q, 2H, J=7Hz, $-CH_2-$), 5.16 (d, 1H, J=5Hz, -CH-), 5.86 (d·d, 1H, J=5Hz, J=9Hz, -CH-), 6~8 (br, 3H, $-NH_3^+$), 6.5~6.8 (m, 1H, =CH-), 6.8 (q, 1H, J=6Hz, -CH-), 6.95 (s, 1H, =CH-), 9.88 (d, 1H, J=9Hz, -CONH-)

Synthesis Example 4

Preparation of 1-(ethoxycarbonyloxy)ethyl 7-[2-(2-tritylaminothiazol-4-yl)-2-ethoxyiminoacetamido]-3-cephem-4-carboxylate (syn isomer)

Procedure A

To a solution of 4.6 g of 2-ethoxyimino-2-(2-tritylaminothiazol-4-yl)acetic acid (syn isomer) in 100 ml of anhydrous methylene chloride, there was added at -20°C, 27 ml of an 8% phosphorus pentachloride-in-methylene chloride solution, and the mixture was stirred at -20°C to -15°C for 30 minutes. Then, 4 ml of triethylamine was added at -20°C and the mixture stirred for 5 minutes. The resulting solution was added to a solution of 3.2 g of 1-(ethoxycarbonyloxy)ethyl 7-amino-3-cephem-4-carboxylate in 100 ml of anhydrous methylene chloride at -25°C, and the mixture stirred at -20°C to -15°C for 30 minutes,

washed with a saturated aqueous sodium hydrogen carbonate solution and then with a saturated aqueous sodium chloride solution, dried and evaporated to dryness under reduced pressure. Addition of isopropyl ether to the residue gave 6.0 g of the title compound as a powder.

Procedure B

Anhydrous methylene chloride (200 ml) was added to 4.6 g of 2-ethoxyimino-2-(2-tritylaminothiazol-4-yl)-acetic acid (syn isomer), 3.2 g of 1-(ethoxycarbonyloxy)-ethyl 7-amino-3-cephem-4-carboxylate and 2.1 g of dicyclohexylcarbodiimide. The mixture was stirred in the suspension state at room temperature for 2 hours and then filtered, the filtrate evaporated to dryness, and the residue dissolved in 200 ml of ethyl acetate. The solution was filtered, washed with a saturated aqueous sodium hydrogen carbonate solution (150 ml x 2) and then with a saturated aqueous sodium chloride solution (150 ml x 2), dried over magnesium sulfate and evaporated to dryness under reduced pressure.

The residue was subjected to silica gel chromatography using ethyl acetate-benzene (1:1 v/v) as the eluant. The eluate fractions containing the desired compound were combined and concentrated under reduced pressure. Addition of isopropyl ether to the residue gave 4.5 g of the title compound as a powder.

Procedure C

In 100 ml of anhydrous methylene chloride was dissolved 4.6 g of 2-ethoxyimino-2-(2-tritylaminothiazol-4-yl)acetic acid (syn isomer), and thereto was added 1.5 ml of triethylamine. Then, an anhydrous methylene chloride solution containing 1 ml of chloro ethyl carbonate was added dropwise at -15°C with stirring. The mixture was stirred at the same temperature for 30 minutes and then cooled to -30°C and a solution of 3.2 g of 1-(ethoxycarbonyloxy)ethyl 7-amino-3-cephem-4-carboxylate in 100 ml of anhydrous methylene

chloride was added dropwise gradually. The resulting mixture was stirred at -25°C to -15°C for an hour and then at -15°C to -10°C for 30 minutes.

The solution was washed with a saturated aqueous sodium hydrogen carbonate solution and then with a saturated aqueous sodium chloride solution, dried over magnesium sulfate and evaporated to dryness under reduced pressure.

The residue was treated in the same manner as Procedure B to give 4.1 g of the title compound.

IR (KBr, $cm^{-1}$): 3300 (br), 1785, 1760, 1680, 1520

NMR ($CDCl_3$, ppm): 1.29 (t, J=7Hz, 3H, $-CH_2CH_3$), 1.33 (t, J=7Hz, 3H, $-CH_2CH_3$), 1.56 (d, J=5Hz, 3H, $-\overset{|}{C}H-CH_3$), 3.25~3.65 (m, 2H, $-SCH_2-$), 4.23 (q, J=7Hz, 2H, $-CH_2CH_3$), 4.36 (q, J=7Hz, 2H, $-CH_2CH_3$), 5.0 (d, J=5Hz, 1H, 6-H), 5.95 (d x d, J=5 and 9Hz, 1H, 7-H), 6.5~6.75 (m, 1H, 3-H), 6.78 (s, 1H, thiazole 5-H), 6.92 (q, J=5Hz, 1H, $-\underset{|}{C}H-CH_3$), 7.39 (s,15H, -NH 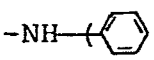 $)_3$), 6.4~8.0 (br, 2H, -CONH- and -NH 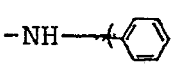 $)_3$),

## Synthesis Example 5

Preparation of 1-(ethoxycarbonyloxy)ethyl 7-[2-(2-aminothiazol-4-yl)-2-ethoxyiminoacetamido]-3-cephem-4-carboxylate (syn isomer) [Compound 3]

The product compound of Synthesis Example 4 (1.5 g) was dissolved in 15 ml of formic acid, and 5 ml of water added thereto. The reaction was allowed to proceed at room temperature for 2 hours. The reaction mixture was then

poured into an appropriate amount of water, the insoluble matter filtered off, and the filtrate made alkaline with sodium hydrogen carbonate and extracted with ethyl acetate. The organic layer was washed with a saturated aqueous sodium chloride solution and dried over sodium sulfate, the ethyl acetate distilled off under reduced pressure, and isopropyl ether added to the residue for crystallization thereof to give 0.82 g of the title compound.

IR (KBr, $cm^{-1}$): 3300 (br), 1760 (br)
1670, 1530

NMR ($(CD_3)_2CO$, ppm):
1.27 (t, J=7Hz, 6H, $-CH_2CH_3$ and $-CH_2CH_3$), 2.54 (d, J=5Hz, 3H, $-\overset{|}{C}H-CH_3$), 3.59~3.89 (m, 2H, $-SCH_2-$), 4.28 (q, J=7Hz, 4H, $-CH_2CH_3$ and $-CH_2CH_3$), 5.17 (d, J=5Hz, 1H, 6-H), 5.99 (d x d, J=5 and 9Hz, 1H, 7-H), 6.5~6.75 (m, 1H, 3-H), 6.73 (s, 1H, thiazole 5-H), 6.9 (q, J=5Hz, 1H, $-\underset{|}{C}H-CH_3$), 6.4~7.7 (br, 2H, $-NH_2$), 8.33 (d, J=9Hz, 1H, -CONH-)

Synthesis Example 6

The product compound of Synthesis Example 5 was treated in the same manner as Synthesis Example 3 to give the title compound of Synthesis Example 5 in the hydrochloride form [Compound 4].

IR (KBr, $cm^{-1}$): 1775, 1765, 1735,
1655, 1545

NMR ($(CD_3)_2SO$, ppm): 1.21 (t, J=7Hz, 3H, $-CH_2CH_3$),

1.26 (t, J=7Hz, 3H, $-CH_2CH_3$), 1.51 (d, J=5Hz, 3H, $-\overset{|}{C}H-CH_3$), 3.4~3.85 (m, 2H, $S-CH_2$), 4.15 (q, J=7Hz, 2H, $-CH_2CH_3$), 4.19 (q, J=7Hz, 2H, $-CH_2CH_3$), 5.13 (d, J=5Hz, 1H, 6-H), 5.86 (d x d, J=5 and 9Hz, 1H, 7-H), 6.5~6.75 (m, 1H, 3-H), 6.75 (q, J=5Hz, 1H, $-\underset{|}{C}H-CH_3$), 6.89 (s, 1H, thiazole 5-H), 5.5~7.9 (br, 3H, $-NH_3^+$), 9.71 (d, J=9Hz, 1H, -CONH-)

Synthesis Examples 7-10

Following Procedure A of Synthesis Example 4 and the procedure of Synthesis Example 5 and/or Synthesis Example 6, the following compounds were obtained:

(c) 1-(Ethoxycarbonyloxy)ethyl 7-[2-(2-aminothiazol-4-yl)-2-isobutoxyiminoacetamido]-3-cephem-4-carboxylate (syn isomer) [Compound 5]

IR (Nujol, $cm^{-1}$): 3300 (br), 1760 (br), 1675, 1525

NMR ($(CD_3)_2CO$, ppm):

0.85 (d, J=6Hz, 6H, $-CH_3x2$), 1.26 (t, J=7Hz, 3H, $-CH_2CH_3$), 1.52 (d, J=5Hz, 3H, $-\overset{|}{C}H-CH_3$), 1.6~2.2 (m, 1H, $-CH_2CH<$), 3.45~3.75 (m, 2H, $-SCH_2-$), 3.8 (d, J=7Hz, 2H, $-CH_2CH<$), 4.25 (q, J=7Hz, 2H, $-CH_2CH_3$), 5.19 (d, J=5Hz, 1H, 6-H), 6.02 (d x d, J=5 and 9Hz, 1H, 7-H), 6.5~6.75 (m, 1H, 3-H), 6.78 (s, 1H, thiazole 5-H), 6.84 (q, J=5Hz, 1H, $-\underset{|}{C}H-CH_3$), 6.4~7.5 (br, 2H, $-NH_2$), 8.3 (d, J=9Hz, 1H, -CONH-)

◎ 1-(Ethoxycarbonyloxy)ethyl 7-[2-(2-aminothiazol-4-yl)-2-n-propoxyiminoacetamido]-3-cephem-4-carboxylate (syn isomer) [Compound 6]

IR (Nujol, $cm^{-1}$): 3300 (br), 1760 (br), 1675, 1530

NMR ($(CD_3)_2CO$, ppm):

0.93 (t, J=7Hz, 3H, $-CH_2CH_2CH_3$), 1.26 (t, J=7Hz, 3H, $-CH_2CH_3$), 1.53 (d, J=5Hz, 3H, $-\overset{|}{C}H-CH_3$), 1.58 (sextet, J=7Hz, 2H, $-CH_2CH_2CH_3$), 3.45~3.8 (m, 2H, $-SCH_2-$), 4.09 (q, J=7Hz, 2H, $-CH_2CH_2CH_3$), 4.18 (q, J=7Hz, 2H, $-CH_2CH_3$), 5.18 (d, J=5Hz, 1H, 6-H), 6.01 (d x d, J=5 and 9Hz, 1H, 7-H), 6.5~6.7 (m, 1H, 3-H), 6.77 (s, 1H, thiazole 5-H), 6.84 (q, J=5Hz, 1H, $-\underset{|}{C}H-CH_3$), 6.2~8.0 (br, 2H, $-NH_2$), 8.38 (d, J=9Hz, 1H, -CONH-)

◎ The hydrochloride of Compound 5 [Compound 7]

IR (Nujol, $cm^{-1}$): 1780, 1760, 1660, 1630, 1545

NMR ($(CD_3)_2SO$, ppm):

0.85 (d, J=6Hz, 6H, $-CH_3$X2), 1.25 (t, J=7Hz, 3H, $-CH_2CH_3$), 1.52, (d, J=5Hz, 3H, $-\underset{|}{C}HCH_3$), 1.7~2.25 (m, 1H, $-CH_2CH$<), 3.4~3.75 (m, 2H, $S-CH_2$), 3.95 (d, J=7Hz, 2H, $-CH_2CH$<), 4.23 (q, J=7Hz, 2H, $-CH_2CH_3$), 5.19 (d, J=5Hz, 1H, 6-H), 5.97 (d x d, J=5 and 9Hz, 1H, 7-H), 6.5~6.75 (m, 1H, 3-H), 6.82 (q, J=5Hz, 1H, $-\underset{|}{C}HCH_3$), 6.95 (s, 1H, thiazole 5-H), 5.5~7.5 (br, 3H, $-NH_3^+$), 9.75 (d, J=9Hz, 1H, -CONH-),

(c) The hydrochloride of Compound 6 [Compound 8]

IR (Nujol, $cm^{-1}$): 1780, 1760, 1655, 1630, 1545

NMR ($(CD_3)_2SO$, ppm):

0.92 (t, J=7Hz, 3H, $-CH_2CH_3$), 1.24 (t, J=7Hz, 3H, $-CH_2CH_3$), 1.53 (d, J=5Hz, 3H, $-CHCH_3$), 1.61 (sextet, J=7Hz, 2H, $-CH_2CH_2CH_3$), 3.4~3.85 (m, 2H, $-SCH_2-$), 4.14 (q, J=7Hz, 2H, $-CH_2CH_2CH_3$), 4.16 (q, J=7Hz, 2H, $-CH_2CH_3$), 5.18 (d, J=5Hz, 1H, 6-H), 5.88 (d x d, J=5 and 9Hz, 1H, 7-H), 6.5~6.8 (m, 1H, 3-H), 6.78 (q, J=5Hz, 1H, $-CHCH_3$), 6.94 (s, 1H, thiazole 5-H). 4.0~7.5 (br, 3H, $-NH_3^+$), 9.76 (d, J=9Hz, 1H, -CONH-),

Synthesis Examples 11-16

Compounds 3, 4, 5, 6, 7 and 8 were prepared by the procedure of Synthesis Example 1 and/or Synthesis Example 3.

Synthesis Example 17

In 300 ml of 0.1% hydrochloric acid was dissolved at room temperature 1g of crude 1-(ethoxycarbonyloxy)ethyl 7-[2-(2-aminothiazol-4-yl)-2-methoxyiminoacetamido]-3-cephem-4-carboxylate hydrochloride (syn isomer). The solution was concentrated at room temperature under reduced pressure until the volume of the solution was reduced to about one fifth of the original. The insoluble matter was filtered off, the filtrate allowed to cool, and the resulting crystalline precipitate collected by filtration and washed with water to give 0.8 g of 1-(ethoxycarbonyloxy)ethyl 7-[2-(2-aminothiazol-4-yl)-2-methoxyiminoacetamido]-3-cephem-4-carboxylate hydrochloride monohydrate (syn isomer) [Compound 9].

Melting point 162-164°C (decomposition).

IR (Nujol, $cm^{-1}$): 3270, 1770, 1760, 1730, 1655, 1260

NMR ($(CD_3)_2SO$, ppm): 1.24 (t, J=7Hz, 3H, $-CH_3$), 1.55 (d, J=6Hz, 3H, $-CH_3$), 3.55~3.8 (m, 2H, $-SCH_2-$), 3.96 (s, 3H, $-OCH_3$), 4.15 (q, J=7Hz, 2H, $-CH_2-$), 5.12 (d, J=5Hz, 1H, $-\overset{|}{C}H-$), 5.17~5.98 (m, 1H, $-\overset{|}{C}H-$), 6.5~6.8 (m, 2H, -CH=, $-\overset{|}{C}H-$), 6.93 (s, 1H, =CH-), 6.0~8.5 (br, 5H, $-NH_3$, $H_2O$), 9.74 (d, J=8Hz, 1H, -CONH)

Elemental analysis:

Calculated for $C_{18}H_{21}N_5O_8S_2 \cdot HCl \cdot H_2O$ (554.00):

C, 39.02%; H, 4.37%; N, 12.64%; Cl, 6.40%;

Found: C, 38.98%; H, 4.11%; N, 12.41%; Cl, 6.67%.

Synthesis Example 18

Preparation of Epimer A in the hydrochloride form [Compound 10]

Five grams (5 g) of 1-(ethoxycarbonyloxy)ethyl (6R,7R)-7-[2-(2-aminothiazol-4-yl)-2-methoxyiminoacetamido]-3-cephem-4-carboxylate (syn isomer) was dissolved in 100 times its weight of 0.5% aqueous hydrochloric acid with heating, the resultant crystalline precipitate (first crop) was collected, and the filtrate concentrated and the resulting crystalline precipitate recrystallized from 50 times its weight of 0.3% aqueous hydrochloric acid. There was thus obtained 1.5 g of pure hydrochloride of Epimer A as colorless needles.

Melting point 156-158°C.

$[\alpha]_D^{25}$ + 43° (C=1, 95% ethanol)

u.v.: $\lambda_{max}$ 236.5nm (95% ethanol)

IR (Nujol, $cm^{-1}$): 1775, 1763, 1730
1655

NMR ($(CD_3)_2SO$, ppm):

1.23 (t, J=7Hz, 3H, $-CH_3$), 1.53(d, J=5.5Hz, 3H, $-CH_3$), 3.62 (m, 2H, $-SCH_2-$), 3.89 (s, 3H, $-OCH_3$), 4.15(q, J=7Hz, 2H, $-CH_2-$), 5.15 (d, J=5.5 Hz, 1H, 6-H), 5.86 (d,d, J=5.5Hz, 8Hz, 1H, 7-H), 4.50~6.80 (br, 3H, $-NH_3^+$), 6.64 (m, 1H, 3-H), 6.78 (q, J=5.5Hz, 1H, -CH-), 6.83 (s, 1H, thiazole 5-H), 9.68 (d, J=8Hz, 1H, -CONH-)

Elemental analysis:

Calculated for $C_{18}H_{22}N_5O_8S_2Cl \cdot 1/2H_2O$ (544.98):

C, 39.67; H, 4.25; N, 12.85; Cl, 6.51%;

Found: C, 39.44; H, 4.30; N, 12.69; Cl, 6.47%.

Synthesis Example 19

Preparation of Epimer A [Compound 11]

To 0.5 g of the product compound of Synthesis Example 18 were added 5 ml of a saturated aqueous sodium hydrogen carbonate solution and 10 ml of ethyl acetate to effect extraction. The organic layer was washed with a saturated aqueous sodium chloride solution and then evaporated to dryness under reduced pressure. Addition of isopropyl ether to the residue gave 0.45 g of Epimer A as a powder.

Melting point 123.0°C-124.0°C

$[\alpha]_D^{26}$ + 48° (C=1, methanol)

IR (KBr, $cm^{-1}$): 1770, 1675

NMR ($CDCl_3$, ppm): 1.29 (t, J=7Hz, 3H, $-CH_2CH_3$),1.56 (d,

J=5.5Hz, 3H, $-\overset{|}{C}H-CH_3$), 3.34~3.70 (m, 2H, $-SCH_2-$), 3.96 (s, 3H, $-OCH_3$), 4.19 (q, J=7Hz, 2H, $-CH_2CH_3$), 5.02 (d, J=5.5Hz, 1H, 6-H), 5.67~6.25 (m, 3H, 7-H and $-NH_2$), 6.46~6.75 (m, 1H, 3-H), 6.61 (s, 1H, thiazole 5-H), 6.82 (q, J=5.5Hz, 1H, $-\overset{|}{C}H-CH_3$), 8.12 (d, J=8Hz, 1H, -CONH-)

Synthesis Example 20

Preparation of Epimer B in the hydrochloride form [Compound 12]

The first crop obtained in Synthesis Example 18 was recrystallized twice from 100 times its weight of 0.5% hydrochloric acid to give 2 g of Epimer B hydrochloride as colorless needles.

Melting point 158-160°C.

$[\alpha]_D^{25}$ + 108°(C=1, 95% ethanol)

u.v : $\lambda_{max}$ 236.5nm (95% ethanol)

IR (Nujol, $cm^{-1}$): 1785, 1730, 1655

NMR ($(CD_3)_2SO$, ppm):

1.22 (t, J=7Hz, 3H, $-CH_2CH_3$), 1.53 (d, J=5.5Hz, 3H, $-\overset{|}{C}H-CH_3$), 3.62 (m, 2H, $-SCH_2-$), 3.89 (s, 3H, $-OCH_3$), 4.15 (q, J=7Hz, 2H, $-CH_2CH_3$), 5.15 (d, J=5.5Hz, 1H, 6-H), 5.86 (d x d, J=5.5Hz and 8Hz, 1H, 7-H), 4.85~7.15 (br, 3H, $-NH_3^+$), 6.64 (m, 1H, 3-H), 6.76 (q, J=5.5Hz, 1H, $-\overset{|}{C}H-CH_3$), 6.85 (s, 1H, thiazole 5-H), 9.73 (d, J=8Hz, 1H, -CONH-)

Elemental analysis:

Calculated for $C_{18}H_{22}N_5O_8S_2Cl \cdot H_2O$ (553.99):

C, 39.02; H, 4.37; N, 12.64; Cl, 6.40%;

Found: C, 39.08; H, 4.21; N, 12.64; Cl, 6.33%.

Synthesis Example 21

Preparation of Epimer B [Compound 13]

Following the procedure of Synthesis Example 19, there was obtained Epimer B from the product compound of Synthesis Example 20.

Melting point 121.0°C-122.0°C.

$[\alpha]_D^{25}$ + 113° (C=1, methanol)

IR (KBr, $cm^{-1}$): 1770, 1670

NMR ($CDCl_3$, ppm): 1.30 (t, J=7Hz, 3H, $-CH_2CH_3$), 1.57 (d, J=5.5Hz, 3H, $-\overset{|}{C}H-CH_3$), 3.30~3.75 (m, 2H, $-SCH_2-$), 3.99 (s, 3H, $-OCH_3$), 4.22 (q, J=7Hz, 2H, $-CH_2CH_3$), 5.06 (d, J=5.5Hz, 1H, 6-H), 5.80 ~6.40 (m, 3H, 7-H and $-NH_2$), 6.45~ 6.75 (m, 1H, 3-H), 6.70 (s, 1H, thiazole 5-H), 6.87 (q, J=5.5Hz, 1H, $-\overset{|}{C}H-CH_3$), 8.15 (d, J=8Hz, 1H, -CONH-)

Synthesis Example 22

Dissolution of 700 mg of 1-(ethoxycarbonyloxy)ethyl (6R,7R)-7-[2-(2-aminothiazol-4-yl)-2-methoxyiminoacetamido]-3-cephem-4-carboxylate [Compound 1] in 10 ml of methanol followed by allowing the solution to stand gave 195 mg of Epimer A [Compound 11].

## Dosage Form Example 1

According to the formula (per tablet) given below, a premix is prepared by mixing Compound 10, tartaric acid and starch, and thereto is added an aqueous hydroxypropylcellulose solution. The mixture is granulated by kneading, drying, crushing and sieving. Another premix prepared from a portion of starch and magnesium stearate is added to and mixed with the granulation product.
The resulting mixture is tableted.

| | |
|---|---|
| Compound 10 | 125 mg (potency) |
| Tartaric acid | 100 mg |
| Magnesium stearate | 2 mg |
| Hydroxypropylcellulose | 2.0 mg |
| Starch in an amount to make the total weight | 300 mg |

## Dosage Form Example 2

Tablets each having the composition shown below are prepared by the procedure of Dosage Form Example 1.

| | |
|---|---|
| Compound 9 | 250 mg (potency) |
| Maleic acid | 100 mg |
| Magnesium Stearate | 2 mg |
| Hydroxypropylcellulose | 2.0 mg |
| Starch in an amount to make the total weight | 500 mg |

## Dosage Form Example 3

Tablets each having the composition shown below are prepared by the procedure of Dosage Form Example 1.

| | |
|---|---|
| Compound 9 | 250 mg (potency) |
| Citric acid | 200 mg |
| Magnesium stearate | 2 mg |
| Hydroxypropylcellulose | 2.0 mg |
| Crystalline cellulose in an amount to make the total weight | 650 mg |

Dosage Form Example 4

Tablets each having the composition shown below are prepared by the procedure of Dosage Form Example 1.

| | |
|---|---|
| Compound 9 | 250 mg (potency) |
| Succinic acid | 250 mg |
| Magnesium stearate | 2 mg |
| Hydroxypropylcellulose | 2.0 mg |
| Starch in an amount to make the total weight | 700 mg |

Dosage Form Example 5

Tablets each having the composition shown below are prepared by the procedure of Dosage Form Example 1.

| | |
|---|---|
| Compound 9 | 250 mg (potency) |
| Tartaric acid | 130.0 mg |
| Magnesium stearate | 2 mg |
| Sucrose fatty acid ester | 50 mg |
| Hydroxymethylcellulose in an amount to make the total weight | 750 mg |

Dosage Form Example 6

Tablets each having the composition shown below are prepared by the procedure of Dosage Form Example 1.

| | |
|---|---|
| Compound 12 | 125 mg (potency) |
| Tartaric acid | 100 mg |
| Magnesium stearate | 2 mg |
| Hydroxypropylcellulose | 2.0 mg |
| Crystalline cellulose in an amount to make the total weight | 300 mg |

Dosage Form Example 7

Tablets each having the composition shown below are prepared by the procedure of Dosage Form Example 1.

| | |
|---|---|
| Compound 1 | 62.5 mg (potency) |
| Maleic acid | 100 mg |
| Magnesium stearate | 2 mg |

| | |
|---|---|
| Hydroxypropylcellulose | 2.0 mg |
| Starch in an amount to make the total weight | 200 mg |

Dosage Form Example 8

Tablets each having the composition shown below are prepared by the procedure of Dosage Form Example 1.

| | |
|---|---|
| Compound 2 | 62.5 mg (potency) |
| Magnesium stearate | 2 mg |
| Hydroxypropylcellulose | 2.0 mg |
| Starch in an amount to make the total weight | 250 mg |

Dosage Form Example 9

Tablets each having the composition shown below are prepared by the procedure of Dosage Form Example 1.

| | |
|---|---|
| Compound 3 | 62.5 mg (potency) |
| Succinic acid | 50 mg |
| Magnesium stearate | 2 mg |
| Hydroxypropylcellulose | 2.0 mg |
| Starch in an amount to make the total weight | 200 mg |

Dosage Form Example 10

Tablets each having the composition shown below are prepared by the procedure of Dosage Form Example 1.

| | |
|---|---|
| Compound 4 | 62.5 mg (potency) |
| Fumaric acid | 50 mg |
| Magnesium stearate | 2 mg |
| Hydroxypropylcellulose | 2 mg |
| Lactose in an amount to make the total weight | 200 mg |

Dosage Form Example 11

Tablets each having the composition shown below are prepared by the procedure of Dosage Form Example 1.

| | |
|---|---|
| Compound 5 | 62.5 mg potency) |
| Magnesium stearate | 2 mg |
| Hydroxypropylcellulose | 2 mg |
| Starch in an amount to make the total weight | 150 mg |

Dosage Form Example 12

Tablets each having the composition shown below are prepared by the procedure of Dosage Form Example 1.

| | |
|---|---|
| Compound 6 | 62.5 mg (potency) |
| Succinic acid | 30.0 mg |
| Magnesium stearate | 2 mg |
| Starch in an amount to make the total weight | 150 mg |

Dosage Form Example 13

Tablets each having the composition shown below are prepared by the procedure of Dosage Form Example 1.

| | |
|---|---|
| Compound 4 | 62.5 mg (potency) |
| Tartaric acid | 50 mg |
| Magnesium stearate | 2 mg |
| Hydroxypropylcellulose | 2.0 mg |
| Starch in an amount to make the total weight | 250 mg |

Dosage Form Example 14

Tablets each having the composition shown below are prepared by the procedure of Dosage Form Example 1.

| | |
|---|---|
| Compound 7 | 62.5 mg (potency) |
| Magnesium stearate | 2 mg |
| Hydroxypropylcellulose | 2.0 mg |
| Starch in an amount to make the total weight | 250 mg |

Dosage Form Example 15

Tablets each having the composition shown below are prepared by the procedure of Dosage Form Example 1.

| | |
|---|---|
| Compound 8 | 62.5 mg (poetncy) |
| Magnesium stearate | 2 mg |
| Hydroxypropylcellulose | 2.0 mg |
| Starch in an amount to make the total weight | 250 mg |

Dosage Form Example 16

In accordance with the formula (per capsule) given below, starch, Compound 12 and succinic acid are added to

and mixed with a premix prepared from a portion of starch and magnesium stearate, and capsules are filled with the resulting mixture in a conventional manner.

| | |
|---|---|
| Compound 12 | 125 mg (potency) |
| Succinic acid | 100 mg |
| Magnesium stearate | 1 mg |
| Starch in an amount to make the total weight | 300 mg |

Dosage Form Example 17

A mixture having the composition (per capsule) shown below is prepared by the procedure of Dosage Form Example 16 and capsules are filled therewith.

| | |
|---|---|
| Compound 11 | 62.5 mg (poetncy) |
| Malic acid | 100 mg |
| Magnesium stearate | 2 mg |
| Lactose in an amount to make the total weight | 200 mg |

Dosage Form Example 18

A mixture having the composition (per capsule) shown below is prepared by the procedure of Dosage Form Example 16 and capsules are filled therewith.

| | |
|---|---|
| Compound 9 | 250 mg (potency) |
| Magnesium stearate | 2 mg |
| Methylcellulose | 20 mg |
| Starch in an amount to make the total weight | 450 mg |

Dosage Form Example 19

A dry sirup is prepared according to the following formula:

| | |
|---|---|
| Compound 9 | 250 mg (potency) |
| Malic acid | 250 mg |
| Sucrose | 170 mg |
| CMC-Na (carboxymethylcellulose sodium) | 20 mg |

Acute Toxicity Test

The acut oral toxicity data for cephalosporin derivatives

(I) and acid addition salt of the present invention in mice are shown below.

Animals: Male mice (ICR, 5-week-old), n=3

Method of administration: The cephalosporin derivatives as prepared in the above synthesis examples were administered in the form of suspension in 1% methylcellulose solution to mice orally at doses of 0.5 g to 5.0 g/kg.

Results:

| Compound | $LD_{50}$ (g/kg) |
|---|---|
| 1 | >5.0 |
| 2 | do. |
| 3 | do. |
| 4 | do. |
| 7 | do. |
| 8 | do. |
| 9 | do. |
| 10 | do. |
| 12 | do. |

## Oral Administration Test 1

The cephalosporin derivatives as prepared in the above synthesis examples were orally administered to humans. The urinary recovery rates found for the unesterified species are shown below in table 1.

Subjects: 3 healthy human adult males

Method of administration: Dosage form preparations containing 62.5 mg (potency) as the unesterified species were orally administered.

Assay: The assay was performed by the paper disc method using *Bacillus subtilis* as the test organism.

Table 1

| Compound No. | Organic acid added | Urinary recovery (%) Hours 0-2 | Hours 2-4 | Hours 4-6 | Total hours 0-6 |
|---|---|---|---|---|---|
| 1 | None | 14.8 | 10.1 | 2.0 | 26.9 |
| 1 | Citric acid | 15.8 | 19.0 | 8.4 | 43.2 |
| 2 | None | 15.8 | 21.1 | 9.1 | 46.0 |
| 2 | Fumaric acid | 20.8 | 21.0 | 9.2 | 51.0 |
| 4 | None | 13.0 | 4.1 | 3.6 | 20.7 |
| 4 | Tartaric acid | 18.0 | 13.4 | 3.1 | 34.5 |

Note 1) Each organic acid was added in an amount of 3 moles per mole of unesterified cephalosporin.

Note 2) The above urinary recovery data are each a mean value for the three subjects.

Oral Administration Test 2

The optically active cephalosporins according to the invention were orally administered to human subjects. The urinary recovery rates found are shown below in Tables 2 and 3.

Method of administration: Dosage form preparations containing 125 mg (potency) as the unesterified species were orally administered by the cross over method.

Assay: The assay was conducted by the paper disc method using *Bacillus subtilis* as the test organism.

Results: As shown in Tables 2 and 3.

Table 2

| Compound No. | Organic acid added | Urinary recovery (%) Hours 0-8 |
|---|---|---|
| 10 | Tartaric acid | 51.1 |
| 10 | - | 41.3 |
| 12 | Tartaric acid | 36.1 |
| 2 | Tartaric acid | 42.4 |

Note 1) The organic acid was added in an amount of 3 moles per mole of the unesterified species.

Note 2) The data are each a mean value for 4 healthy adults.

Table 3

| Compound No. | Organic acid added | Urinary recovery (%) Hours 0-8 |
|---|---|---|
| 11 | Tartaric acid | 45.0 |
| 11 | - | 35.8 |
| 13 | Tartaric acid | 30.6 |
| 2 | Tartaric acid | 35.2 |

Note 1) The organic acid was added in an amount of 3 moles per mole of the unesterified species.

Note 2) The data are each a mean value for 4 healthy adults.

Oral Administration Test 3

The cephalosporin preparations according to the invention were orally administered to human subjects. The blood level and urinary recovery rate data found for the unesterified species are shown below in Table 4.

Method of administration: The dosage form preparations prepared in the dosage form examples specified in Table 4 were administered orally to adult males.

Assay: The assay was carried out by the paper disc method using Bacillus subtilis as the test organism.

Results: As shown in Table 4.

Table 4

| Dosage Form | Blood level (mcg/ml) after | | | | | Urinary recovery % |
|---|---|---|---|---|---|---|
| Example No. | 0.5 | 1 | 2 | 4 | 6 hours | (Hours 0-24) |
| 2 | 2.7 | 3.8 | 3.6 | 1.9 | 0.9 | 45.0 |
| 3 | 3.0 | 3.2 | 3.0 | 1.9 | 0.8 | 43.5 |
| 4 | 2.0 | 3.4 | 2.5 | 2.0 | 1.0 | 42.0 |
| 5 | 3.2 | 4.0 | 3.5 | 2.0 | 1.0 | 50.8 |
| 18 | 2.0 | 3.2 | 3.0 | 1.4 | 0.4 | 38.9 |
| 19 | 3.8 | 4.2 | 3.1 | 1.8 | 1.0 | 50.6 |

Oral Administration Test 4

The dosage forms prepared according to the basic formulas given below were orally administered to human subjects. The urinary recovery data found for the unesterified species are shown below in Table 5. The method of administration and the assay method were the same as in Oral Administration Test 3.

Basic formula 1 (Tablet)

| | |
|---|---|
| Cephalosporin derivative (I) or salt thereof | 125 mg (potency) |
| Tartaric acid | 0 mg or 125 mg |
| Magnesium stearate | 2 mg |
| Hydroxypropylcellulose | 3 mg |
| Starch | Proper quantity |

Basic formula 2 (Tablet)

| | |
|---|---|
| Cephalosporin derivative (I) or salt therof | 250 mg (potency) |
| Citric acid | 0 mg or 150 mg |
| Sodium hydrogen carbonate | 25 mg |
| Lactose | Proper quantity |

Basic formula 3 (Capsule)

| | |
|---|---|
| Cephalosporin derivative (I) or salt thereof | 125 mg (potency) |
| Tartaric acid | 0 mg or 125 mg |
| Magnesium stearate | 3 mg |
| Hydroxypropylcellulose | 5 mg |
| Lactose | Proper quantity |

Basic formula 4 (Capsule)

| | |
|---|---|
| Cephalosporin derivative (I) or salt thereof | 125 mg (potency) |
| Malic acid | 0 mg or 125 mg |
| Talc | 3 mg |
| Hydroxypropylcellulose | 3 mg |
| Starch | Proper quantity |

Basic formula 5 (Capsule)

| | |
|---|---|
| Cephalosporin derivative (I) or salt thereof | 125 mg (potency) |
| Succinic acid | 0 mg or 50 mg |
| Sucrose ester | 30 mg |
| Crystalline cellulose | Proper quantity |

Basic formula 6 (Capsule)

| | |
|---|---|
| Cephalosporin derivative (I) or salt thereof | 125 mg (potency) |
| Fumaric acid | 0 mg or 125 mg |
| Crystalline cellulose | 10 mg |
| Magnesium stearate | 2 mg |
| Lactose | Proper quantity |

Basic formula 7 (Capsule)

| | |
|---|---|
| Cephalosporin derivative (I) or salt therof | 125 mg (potency) |
| Tartaric acid | 0 mg or 125 mg |
| Sodium hydrogen carbonate | 15 mg |
| Magnesium stearate | 3 mg |
| Starch | Proper quantity |

Table 5

| Basic formula No. | Compound No. | Organic acid | Urinary recovery (%) Hours 0-8 |
|---|---|---|---|
| 1 | 1 | - | 18.8 |
| 2 | 1 | - | 9.5 |
| 1 | 1 | Tartaric acid | 20.9 |
| 2 | 1 | Citric acid | 29.8 |
| 1 | 2 | - | 37.9 |
| 2 | 9 | - | 31.5 |
| 1 | 2 | Tartaric acid | 43.2 |
| 2 | 9 | Citric acid | 45.8 |
| 3 | 1 | - | 20.0 |
| 4 | 11 | - | 22.5 |
| 7 | 11 | - | 25.1 |
| 3 | 1 | Tartaric acid | 27.6 |
| 4 | 11 | Malic acid | 35.6 |
| 7 | 11 | Tartaric acid | 31.0 |
| 3 | 9 | - | 37.2 |
| 4 | 12 | - | 45.6 |
| 7 | 12 | - | 40.0 |
| 3 | 9 | Tartaric acid | 42.8 |
| 4 | 12 | Malic acid | 56.2 |
| 7 | 12 | Tartaric acid | 55.2 |
| 6 | 4 | Maleic acid | 41.5 |
| 5 | 8 | Succinic acid | 40.5 |

What we claimed is:

1. A compound of the formula

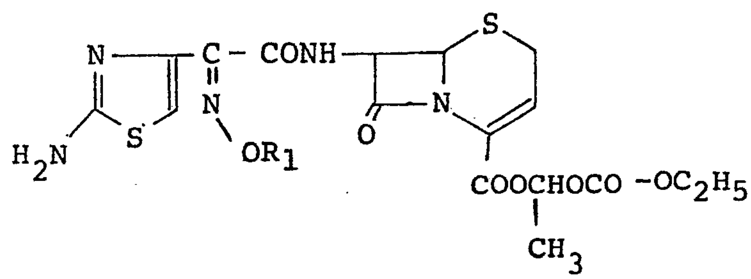

wherein $R_1$ is a lower alkyl, or its pharmaceutically acceptable acid addition salt.

2. The compound according to claim 1 which is in the form of hydrochloride.
3. The compound according to claim 1 which is the syn isomer of 1-(ethoxycarbonyloxy)ethyl 7-[2-(2-aminothiazol-4-yl)-2-methoxyiminoacetamido]-3-cephem-4-carboxylate.
4. The compound according to claim 1 which is the syn isomer of 1-(ethoxycarbonyloxy)ethyl 7-[2-(2-aminothiazol-4-yl)-2-methoxyiminoacetamido]-3-cephem-4-carboxylate hydrochloride.
5. The compound according to claim 4 which is in the form of crystalline hydrate.
6. The compound according to claim 4 which is in the form of crystalline monohydrate.
7. The compound according to claim 3 which is optically active and has the specific rotation of $[\alpha]_D^{26} + 48°$ (c=1, methanol).
8. The compound according to claim 4 which is optically active and has the specific rotation of $[\alpha]_D^{25} + 43°$ (c=1, 95% ethanol).
9. The compound according to claim 1 which is the syn isomer of 1-(ethoxycarbonyloxy)ethyl 7-[2-(2-aminothiazol-4-yl)-2-ethoxyiminoacetamido]-3-cephem-4-carboxylate hydrochloride.

10. The compound according to claim 1 which is the syn isomer of 1-(ethoxycarbonyloxy)ethyl 7-[2-(2-aminothiazol-4-yl)-2-isobutoxyiminoacetamido]-3-cephem-4-carboxylate hydrochloride.

11. The compound according to claim 1 which is the syn isomer of 1-(ethoxycarbonyloxy)ethyl 7-[2-(2-aminothiazol-4-yl)-2-n-propoxyiminoacetamido]-3-cephem-4-carboxylate hydrochloride.

12. An orally administrable pharmaceutial composition for treatment of infectious diseases caused by bacteria, comprising as an active ingredient a compound of the formula

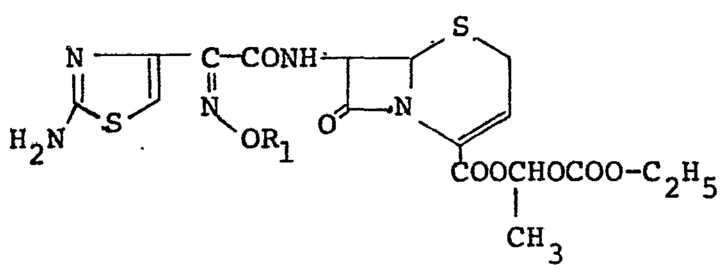

wherein $R_1$ is a lower alkyl or its pharmaceutucally acceptable acid addition salt.

13. The pharmaceutical composition according to claim 12, wherein the acid addition salt is hydrochloride.

14. The pharmaceutical composition according to claim 12, wherein the active ingredient is the syn isomer of 1-(ethoxycarbonyloxy)ethyl 7-[2-(2-aminothiazol-4-yl)-2-methoxyiminoacetamido]-3-cephem-4-carboxylate.

15. The pharmaceutical composition according to claim 12, wherein the active ingredient is the syn isomer of 1-(ethoxycarbonyloxy)ethyl 7-[2-(2-aminothiazol-4-yl)-2-methoxyiminoacetamido]-3-cephem-4-carboxylate hydrochloride.

16. The pharmaceutical composition according to claim 15, wherein the active ingredient is in the form of crystalline hydrate.

17. The pharmaceutical composition according to claim 15, wherein the active ingredient is in the form of crystalline monohydrate.
18. The pharmaceutical composition according to claim 14, wherein the active ingredient is optically active and has the specific rotation of $[\alpha]_D^{26}$+48° (c=1, methanol)
19. The pharmaceutical composition according to claim 15, wherein the active ingredient is optically active and has the specific rotation of $[\alpha]_D^{25}$+ 43° (c=1, 95% ethanol)
20. The pharmaceutical composition according to claim 12, wherein the active ingredient is the syn isomer of 1-(ethoxycarbonyloxy)ethyl 7-[2-(2-aminothiazol-4-yl)-2-ethoxyiminoacetamido]-3-cephem-4-carboxylate hydrochloride.
21. The pharmaceutical composition according to claim 12, wherein the active ingredient is the syn isomer of 1-(ethoxycarbonyloxy)ethyl 7-[2-(2-aminothiazol-4-yl)-2-isobutoxyiminoacetamido]-3-cephem-4-carboxylate hydrochloride.
22. The pharmaceutical composition according to claim 12, wherein the active ingredient is the syn isomer of 1-(ethoxycarbonyloxy)ethyl 7-[2-(2-aminothiazol-4-yl)-2-n-propoxyiminoacetamido]-3-cephem-4-carboxylate hydrochloride.
23. The pharmaceutical composition according to any of claims 12 - 22, which also contains an organic acid.
24. The pharmaceutical composition according to Claim 23, wherein the organic acid is an aliphatic carboxylic acid which contains at least two carboxyl groups.
25. The pharmaceutical composition according to Claim 23, wherein the aliphatic carboxylic acid is a hydroxy acid.
26. The pharmaceutical composition according to any of Claims 23-25, which is in the form of a solid preparation.
27. The pharmaceutical composition according to Claim 25, which also contains at least one member selected from the group consisting of alkali metal carbonates and alkali metal hydrogen carbonates.

28. A method of preparing orally administrable compositions for the treatment of bacteria-caused infectious diseases, which comprises mixing a cephalosporin derivative of the general formula:

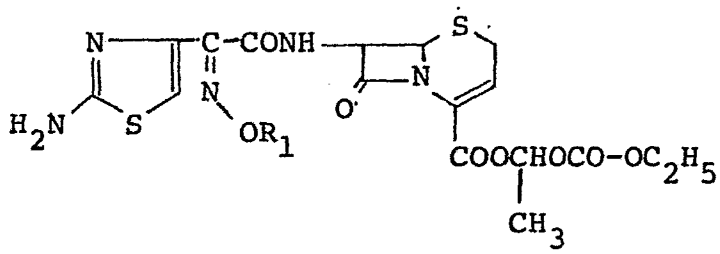

wherein $R_1$ is a lower alkyl group or a acid addition salt thereof with an organic acid.

29. A method of producing cephalosporin derivatives of the general formula

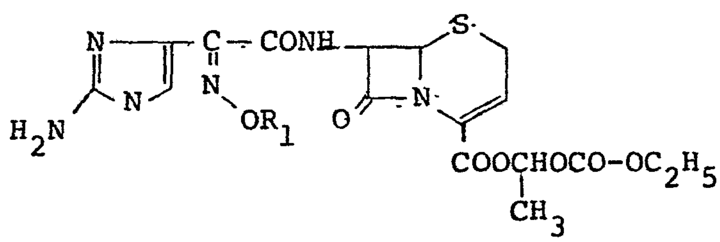

wherein $R_1$ is a lower alkyl group, pharmaceutically acceptable acid addition salts thereof, and crystalline hydrates of these, which comprises reacting a compound of the general formula

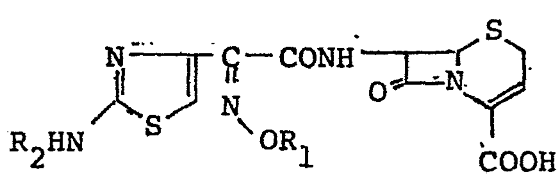

wherein $R_1$ is as defined above and $R_2$ is H or an amino-protecting group, or a reactive derivative thereof with a compound of the general formula

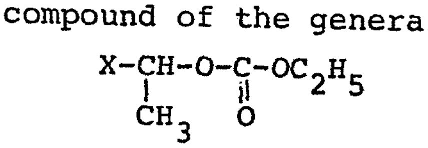

wherein X is an atom or group which is reactive with the carboxyl group or a reactive derivative thereof, eliminating the protective group $R_2$ if $R_2$ is such, or reacting a compound of the general formula

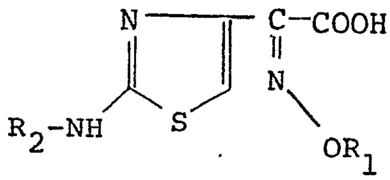

wherein $R_1$ and $R_2$ are as defined above,

or a reactive derivative thereof with a compound of the general formula

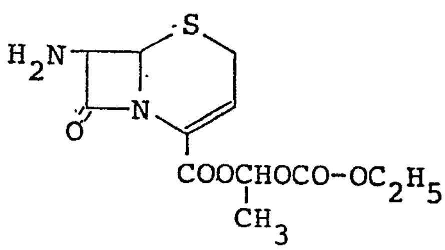

optionally converting the product to an acid addition salt, and optionally recrystallizing the final product from an aqueous solvent, aqueous hydrochloric acid or an organic solvent.